# EUROPÄISCHE PATENTSCHRIFT

(11) **EP 2 823 828 B1**
(45) Veröffentlichungstag und Bekanntmachung des Hinweises auf die Patenterteilung: **21.12.2016**
(21) Anmeldenummer: 14002219.5
(22) Anmeldetag: 28.06.2014
(51) Int. Cl.: A61L 2/26, B08B 3/04

(54) **Vorrichtung zur Behandlung von kleineren Gegenständen**
Device for treating small objects
Dispositif de traitement de petits objets

(30) Priorität: 08.07.2013 DE 102013011287
(43) Veröffentlichungstag der Anmeldung: 14.01.2015
(73) Patentinhaber: Atec Pharmatechnik Gmbh, 24966 Sörup (DE)
(72) Erfinder: Mumm, Hans-Werner, 24966 Sörup (DE)
(74) Vertreter: Thomas, Götz

(56) Entgegenhaltungen:
- EP-A2- 0 661 062
- EP-B1- 1 449 543
- EP-B1- 1 769 889
- DE-A1- 4 018 023
- US-B1- 6 835 362

## Beschreibung

Die Erfindung betrifft eine Vorrichtung zur Behandlung von kleineren Gegenständen, insbesondere zur Reinigung und Sterilisation von Spritzenkolben oder Verschlüssen von Spritzen, Infusionsflaschen und Vials für pharmazeutische Zwecke, mit einem Behälter zur Aufnahme einer Mehrzahl der Gegenstände, einer Halterung, die gemeinsam mit dem Behälter motorisch um eine horizontale Schwenkachse schwenkbar ist, sowie zwei vom Behälter aus durch die Halterung und eine ortsfeste Wand verlaufenden Rohrleitungen zur Umwälzung eines Behandlungsmediums durch den Behälter. Die Vorrichtung ist besonders für eine Reinraumumgebung bzw. zur Anbringung an der Innenseite einer Wand eines Reinraums bestimmt.
Vorrichtungen der eingangs genannten Art zum Reinigen und Sterilisieren von kleineren Gegenständen sind aus der EP 1 449 543 B2 des Anmelders sowie aus der WO 00/61199 A1 bekannt, in denen auch der Verwendungszweck dieser Vorrichtungen näher erläutert wird. Der Behälter dient dort auch zum Transport der zu reinigenden bzw. der sterilisierten Gegenstände innerhalb eines Reinraums.

Die DE 44 09 659 A1 und die DE 27 39 169 A1 offenbaren ähnliche Vorrichtungen, bei denen der Behälter während der Behandlung der Gegenstände jedoch nicht verschwenkt werden kann, um diese zur Verbesserung der Reinigungs- und Sterilisationswirkung im Behälter in Bewegung zu versetzen. Der an einer ortsfesten Wand gehalterte Behälter gemäß der DE 44 09 659 A1 kann direkt in eine Übergabestation entleert werden während der Behälter gemäß der DE 27 39 169 A1 durch einen durch die Wand hindurch verlaufenden, oberhalb des Behälters angeordneten Stutzen mit den kleinen Gegenständen befüllt werden kann, wodurch die Handhabung erleichtert und mit weniger Behältern kürzere Zykluszeiten erzielt werden können. Jedoch ist zum Entleeren bzw. zum Befüllen der bekannten Behälter in der Vorrichtung eine große Raumhöhe erforderlich.

Weiter ist es aus der DE 40 18 023 A1 bei einer Vorrichtung zur Sterilisation von Gegenständen, insbesondere pharmazeutischen Verschlusselementen, bekannt, in einer Autoklavenkammer eine drehbare Reinigungstrommel durch eine zur Drehachse der Trommel koaxiale Antriebs-Hohlwelle anzutreiben, die zugleich auch zur Zufuhr von Behandlungsmedien dient. Die Antriebs-Hohlwelle ist dicht und drehbar durch eine Seitenwand der Autoklavenkammer zu einer Rohrleitungskupplung geführt, wo sie lösbar mit einer in die Trommel führenden, mit Spritzdüsen versehenen Verteiler-Hohlwelle für die Behandlungsmedien verbunden ist.

Darüber hinaus offenbart die EP 1 769 889 B1 des Anmelders eine Vorrichtung zum Heben und Drehen von Behältern in einer Reinraumumgebung, die einen Schwenk- oder Hubarm mit einer um eine horizontale Drehachse drehbaren Halterung zur Aufnahme eines Behälters umfasst.

Ausgehend hiervon liegt der Erfindung die Aufgabe zugrunde, eine Vorrichtung der eingangs genannten Art dahingehend zu verbessern, dass ein in der Halterung gehalterter Behälter ohne die Notwendigkeit einer großen Raumhöhe direkt aus einer Befüllstation befüllt und/oder direkt in eine Übergabestation entleert werden kann. Vorteilhaft soll der Behälter bei Bedarf auch weiterhin abgenommen und als Transportbehälter genutzt werden können.

Diese Aufgabe wird bei der erfindungsgemäßen Vorrichtung dadurch gelöst, dass die Halterung an einem Schwenk- oder Hubarm angebracht ist, der in Bezug zur Wand motorisch um eine zur Schwenkachse parallele zweite Schwenkachse schwenkbar ist, und dass die durch den Schwenk- oder Hubarm hindurch verlaufenden Rohrleitungen zwischen der Wand und dem Schwenk- oder Hubarm eine Rohrleitungskupplung umfassen.

Der Schwenk- oder Hubarm gestattet es, den Behälter anzuheben, abzusenken und dabei um die horizontale Schwenkachse in Bezug zum Schwenk- oder Hubarm zu drehen, wodurch sich eine Befüll- und/oder Entleeröffnung des Behälters innerhalb der Reichweite des Schwenk- oder Hubarms je nach Bedarf an eine Befüll- und/ oder eine Entleerstation andocken lässt, so dass eine Entnahme des Behälters aus der Halterung zum Befüllen und/oder zum Entleeren nicht erforderlich ist. Jedoch ist der Behälter bevorzugt lösbar mit der Halterung verbunden, so dass er bei Bedarf auch abgenommen und als Transportbehälter zum Transport der zu reinigenden und/oder der behandelten Gegenstände genutzt werden kann.

Bei der Vorrichtung ist die Rohrleitungskupplung bevorzugt zwischen ersten, durch die Wand verlaufenden Abschnitten und zweiten, durch den Schwenk- oder Hubarm verlaufende Abschnitten der Rohrleitungen angeordnet, wobei es die Kupplung gestattet, die ersten und die zweiten Rohrleitungsabschnitte gas- und/oder flüssigkeitsdicht miteinander zu verbinden, wenn diese sich in einer definierten Behandlungsstellung des Schwenk- oder Hubarms gegenüberliegen, um zur Behandlung von kleinen Gegenständen im Behälter ein Behandlungsmedium, wie Wasser, Dampf oder Heißluft aus einer jenseits der Wand befindlichen Medienquelle durch die Rohrleitungen und den Behälter umzuwälzen.

Dabei sieht eine bevorzugte Ausgestaltung der Erfindung vor, dass die durch den Schwenk- oder Hubarm verlaufenden zweiten Rohrleitungsabschnitte beim Verschwenken des Behälters um die Schwenkachse mit diesem und der Halterung mitgeschwenkt werden, und dass bei geschlossener Rohrleitungskupplung die durch die Wand hindurch verlaufenden und in Bezug zur Wand drehbar gelagerten ersten Rohrleitungsabschnitte drehfest mit den zweiten Rohrleitungsabschnitten gekuppelt sind, so dass sie mit diesen mitgeschwenkt werden.

Zum anderen gestattet es die Rohrleitungskupplung auch, die durch die Wand verlaufenden ersten Rohrleitungsabschnitte und die durch den Schwenk- oder Hubarm verlaufenden zweiten Rohrleitungsabschnitte voneinander zu trennen, wenn der Schwenk- oder Hubarm zum Befüllen und/oder Entleeren des Behälters verschwenkt werden soll, was eine seitliche Relativbewegung zwischen den ersten und den zweiten Rohrleitungsabschnitten notwendig macht.

Durch den Verlauf der Rohrleitungen durch den Schwenk- oder Hubarm ist es möglich, den Schwenk- oder Hubarm zwischen der Wand und der Halterung anzuordnen und die zweiten Abschnitte der Rohrleitungen in der Behandlungsstellung entlang der Schwenkachse des Behälters geradlinig zur Wand und zu den gegenüberliegenden ersten Abschnitten zu führen.

Das Verbinden der gegenüberliegenden Rohrleitungsabschnitte mit Hilfe der Rohrleitungskupplung wird nachfolgend auch als Kuppeln oder Schließen der Kupplung bezeichnet, während das Trennen der gegenüberliegenden Rohrleitungsabschnitte nachfolgend auch als Abkuppeln oder Trennen der Kupplung bezeichnet wird.

Wenn die erfindungsgemäße Vorrichtung in einer Reinraumumgebung verwendet wird, handelt es sich bei der ortsfesten Wand um eine Begrenzungswand des Reinraums, wobei der Schwenk- oder Hubarm, die Halterung und der Behälter innerhalb des Reinraums und ein Maschinenteil der Vorrichtung sowie mindestens eine Quelle für das Behandlungsmedium jenseits der Wand, d.h. außerhalb des Reinraums angeordnet ist.

Eine vorteilhafte Ausgestaltung der Erfindung sieht vor, dass der Schwenk- oder Hubarm an oder nahe seinem einen Ende durch ein erstes Drehgelenk mit der Wand verbunden ist und an seinem entgegengesetzten Ende die Halterung für den Behälter trägt, die durch ein zweites Drehgelenk drehbar mit dem Schwenk- oder Hubarm verbunden ist. Beide Drehgelenke weisen parallele horizontale Drehachsen auf.

Der Schwenk- oder Hubarm und die Halterung werden jeweils mittels Drehantrieben verschwenkt, wobei ein erster Drehantrieb zum Verschwenken des Schwenk- oder Hubarms zweckmäßig jenseits der Wand angeordnet ist, während ein zweiter Drehantrieb zum Verschwenken der Halterung zweckmäßig im Inneren des Schwenk- oder Hubarms angeordnet ist. Die zur Energieversorgung des zweiten Drehantriebs notwendigen Leitungen verlaufen bevorzugt durch eine Drehdurchführung des ersten Drehgelenks, während die Rohrleitungen bzw. die zweiten Rohrleitungsabschnitte bevorzugt durch eine Drehdurchführung des zweiten Drehgelenks verlaufen. Dadurch kann die Halterung in jeder Stellung des Schwenk- oder Hubarms in Bezug zu diesem um die horizontale Schwenkachse geschwenkt werden. Zweckmäßig besitzen die beiden Drehantriebe die gleiche Bauart, um die Wartung zu vereinfachen und die Anzahl der verschiedenen Bauteile der Vorrichtung zu verringern. Beide Drehantriebe sind vorteilhaft mit einem selbsthemmenden Untersetzungsgetriebe ausgestattet, so dass sich der Schwenk- oder Hubarm und die Halterung bei einem Stromausfall nicht nach unten bewegen können.

Gemäß einer weiteren bevorzugten Ausgestaltung der Erfindung sind die ersten Rohrleitungsabschnitte in Bezug zur Wand um eine in der Behandlungsstellung mit der Schwenkachse fluchtende horizontale Drehachse drehbar. Vorteilhaft verlaufen die ersten Rohrleitungsabschnitte gemeinsam durch einen zur Drehachse allgemein koaxialen Drehkörper, der in Bezug zur ortsfesten Wand drehbar ist. Die Rohrleitungsabschnitte sind dabei zweckmäßig in gleichen radialen Abständen von der Drehachse angeordnet.

Das Kuppeln und Trennen der jeweils gegenüberliegenden ersten und zweiten Rohrleitungsabschnitte erfolgt bevorzugt durch eine Relativbewegung, bei der einer der jeweils gegenüberliegenden Rohrleitungsabschnitte in der Behandlungsstellung in einer zur Drehachse bzw. zur ersten und zweiten Schwenkachse parallelen Richtung an den anderen der Rohrleitungsabschnitte angenähert bzw. von diesem weg bewegt wird. Vorteilhaft sind die ersten Rohrleitungsabschnitte dazu drehfest mit einem Schiebekörper verbunden, der zusammen mit den ersten Rohrleitungsabschnitten im Drehkörper entlang von dessen Drehachse verschiebbar ist, um die ersten Rohrleitungsabschnitte zum Kuppeln auf die zweiten Rohrleitungsabschnitte zu oder zum Abkuppeln von diesen weg zu bewegen. Der Schiebekörper ist zweckmäßig drehfest mit dem Drehkörper verbunden, so dass er beim Drehen der Drehkörpers mit diesem mitgedreht wird.

Wenn bei geöffneter Kupplung die ersten und die zweiten Rohrleitungsabschnitte voneinander getrennt sind, stehen die ersten Rohrleitungsabschnitte vorteilhaft nicht über eine der Halterung zugewandte Oberfläche der Wand über, so dass das Verschwenken des Hub- oder Schwenkarms nicht behindert wird.

Damit sich die ersten und die zweiten Rohrleitungsabschnitte in der Behandlungsstellung miteinander kuppeln lassen, fluchten in der Behandlungsstellung vorteilhaft nicht nur die zweiten Rohrleitungsabschnitte mit den ersten Rohrleitungsabschnitten sondern auch die Schwenkachse des Behälters mit der Drehachse der ersten Rohrleitungsabschnitte und des Drehkörpers.

Um sicherzustellen, dass die ersten Rohrleitungsabschnitte mit den zweiten Rohrleitungsabschnitten fluchten, wenn der Hub- oder Schwenkarm mit dem Behälter in die Behandlungsstellung bewegt wird, werden die ersten Rohrleitungsabschnitte beim Trennen der Rohrleitungskupplung zweckmäßig bis zum erneuten Schließen der Kupplung in einer definierten Drehstellung verriegelt. Dazu dient eine Verriegelungseinrichtung, mit der sich zweckmäßig der Drehkörper drehfest mit der Wand oder einem den Drehkörper umgebenden ortsfesten Gehäuse verriegeln lässt.

Vorteilhaft umfasst die Verriegelungseinrichtung einen Riegel und eine Riegelaufnahme, die sich mittels eines Stellorgans durch eine zur Schwenkachse parallele Relativbewegung in Eingriff miteinander bringen lassen, wobei zweckmäßig entweder der Riegel drehfest mit dem Drehkörper und die Riegelaufnahme drehfest mit der Wand verbunden ist, oder umgekehrt. Um eine genaue Positionierung zu erreichen, weist die Riegelaufnahme in Bewegungsrichtung des Riegels verjüngte Führungsflächen auf, die eine Zentrierung des Riegels in der Riegelaufnahme bewirken.

Zweckmäßig weist einer von den jeweils gegenüberliegenden Rohrleitungsabschnitten an seinem Ende ein Kupplungselement auf, das beim Kuppeln in eine komplementäre Vertiefung in einem Kupplungselement am Ende des jeweils gegenüberliegenden Rohrleitungsabschnitts eintritt. Dadurch kann nicht nur die Dichtigkeit der Verbindung verbessert werden, sondern zwischen den beiden gegenüberliegenden Rohrleitungsabschnitten auch eine drehfeste formschlüssige Verbindung hergestellt werden. Auf diese Weise drehen sich die ersten Rohrleitungsabschnitte immer mit, wenn die zweiten Rohrleitungsabschnitte in der Behandlungsstellung zusammen mit dem Behälter durch dessen Drehantrieb verschwenkt werden.

Zur Abdichtung der Rohrleitungsabschnitte im Bereich der Rohrleitungskupplung ist zwischen den zusammenwirkenden Kupplungselementen mindestens eine Dichtung angeordnet, die beim Kuppeln durch die axiale Relativbewegung gegen eine gegenüberliegende Dichtfläche angepresst wird.

Im Folgenden wird die Erfindung anhand eines in der Zeichnung dargestellten Ausführungsbeispiels näher beschrieben.
Fig. 1 zeigt eine perspektivische Ansicht von Teilen eines Reinraums mit einer erfindungsgemäßen Vorrichtung zur Reinigung und Sterilisation von kleinen Gegenständen, die einen Schwenk- oder Hubarm und eine daran angebrachte motorisch schwenkbare Halterung zur Aufnahme eines Behandlungs- und/oder Transportbehälters umfasst, während des Anbringens des Behälters an der Halterung;
Fig. 2 zeigt eine entsprechende Ansicht, jedoch nach dem Anbringen des Behälters an der Halterung;
die Figuren 3 und 4 zeigen entsprechende Ansichten, jedoch während der Reinigung und Sterilisation der Gegenstände im Behälter in unterschiedlichen Schwenkstellungen des Behälters und der Halterung;
Fig. 5 zeigt eine entsprechende Ansicht, jedoch nach dem Andocken des Behälters an eine Andocköffnung einer Befüllstation;
Fig. 6 zeigt eine teilweise geschnittene Seitenansicht eines Teils der Vorrichtung bei abgenommenem Behälter;
Fig. 7 zeigt eine perspektivische Ansicht von Teilen der Vorrichtung mit einer zwischen dem Schwenk- oder Hubarm und einer Begrenzungswand des Reinraums angeordneten Rohrleitungskupplung zwischen Abschnitten von Rohrleitungen zum Umwälzen eines Behandlungsmediums durch den Behälter, in geschlossenem Zustand der Rohrleitungskupplung;
Fig. 8 zeigt eine andere perspektivische Ansicht der Teile der Vorrichtung aus Fig. 8, jedoch in geöffnetem Zustand der Rohrleitungskupplung;
Fig. 9 zeigt eine schematische Schnittansicht von Teilen eines ersten und eines gegenüberliegenden zweiten Rohrleitungsabschnitts im Bereich der geöffneten Kupplung;
Fig. 10 zeigt eine Schnittansicht entlang der Linie X-X der Fig. 6.

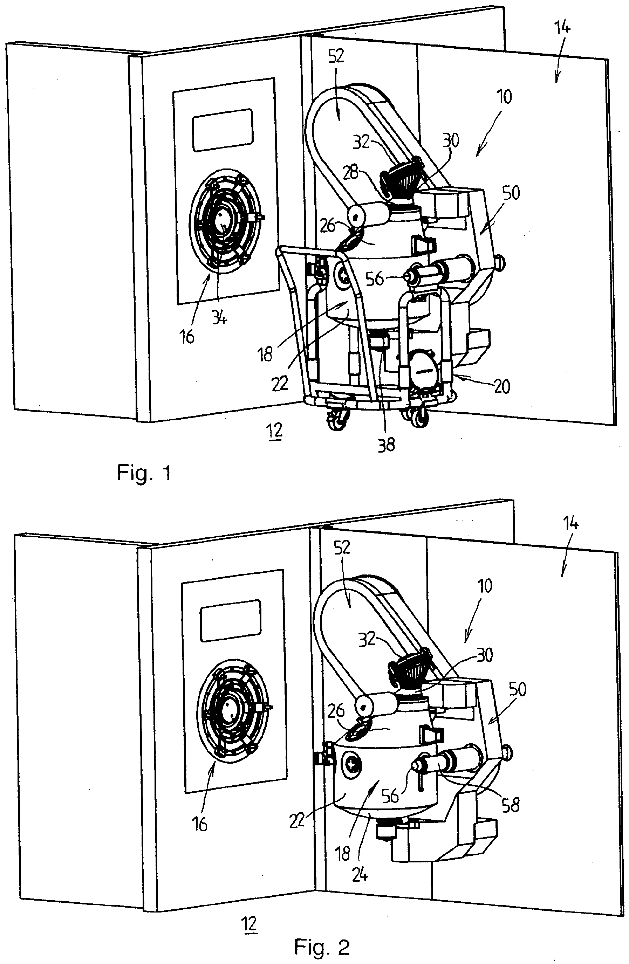

Die in der Zeichnung dargestellte Vorrichtung 10 ist in einem Reinraum 12 an der Innenseite einer Begrenzungswand 14 des Reinraums 12 angebracht und dient zur Reinigung, Sterilisation und ggf. zur Silikonisierung von kleinen Gegenständen, wie z.B. Verschlüssen für pharmazeutische Verpackungsbehälter, die aus einer neben der Vorrichtung 10 an der Begrenzungswand 14 angeordneten Befüllöffnung 16 einer Befüllstation in einen Behandlungs- und/oder Transportbehälter 18 eingebracht und mit Hilfe der Vorrichtung 10 innerhalb des Behälter 18 gereinigt, sterilisiert und ggf. silikonisiert werden. Alternativ können die Gegenstände jedoch auch an anderer Stelle in den Behälter 18 gefüllt werden, bevor dieser mit Hilfe eines fahrbaren Behältertransportwagens 20 zur Vorrichtung 10 transportiert wird, wie in Fig. 1 dargestellt.

Nach der Behandlung der Gegenstände innerhalb des Behälters 18 kann dieser entweder aus der Vorrichtung 10 entnommen und mittels des Behältertransportwagens 20 zu einer entfernten Übergabestation (nicht dargestellt) transportiert werden, oder alternativ in eine Entleeröffnung (nicht dargestellt) einer benachbarten Übergabestation entleert werden, die ähnlich wie die Befüllöffnung 16 neben der Vorrichtung 10, zum Beispiel an deren anderer Seite, an der Begrenzungswand 14 angeordnet ist. Aus der Übergabestation können die Gegenstände dann in sterilem Zustand entnommen und weiterverarbeitet werden.

Wegen der hohen Anforderungen an die Reinheit erfolgen sowohl die Behandlung der Verschlüsse mittels der Reinigungs- und Vorrichtung 10 sowie das Befüllen und Entleeren des Behälters 18 innerhalb des Reinraums 12.

Wie in den Figuren 1 bis 5 dargestellt, weist der Behälter 18 in aufrechter Stellung ein im Wesentlichen zylindrisches Unterteil 22 mit einem konvex nach unten gewölbten Boden 24 und ein nach oben zu verjüngtes Oberteil 26 auf. Am oberen Ende des Oberteils 26 befindet sich eine durch ein Klappenventil 28 verschließbare Öffnung 30 mit größerem Öffnungsquerschnitt, durch welche die zu reinigenden und zu sterilisierenden kleinen Gegenstände in den Behälter 18 eingefüllt werden und durch welche die gereinigten und sterilisierten Gegenstände entleert werden, wenn der Behälter 18 in umgedrehtem Zustand an die Übergabestation angekoppelt ist und das Ventil 28 geöffnet wird. Die Öffnung 30 ist in Bezug zu Längsachse des Unterteils 22 nach einer Seite versetzt. Jenseits des Klappenventils 28 ist ein Beta-Teil 32 eines Rapid-Transfer-Ports der Firma SNE La Calhene, Frankreich angebracht, der zum Kuppeln mit einem komplementären Alpha-Teil 34 an der Befüllöffnung 16 bzw. der Entleeröffnung der Übergabestation dient.

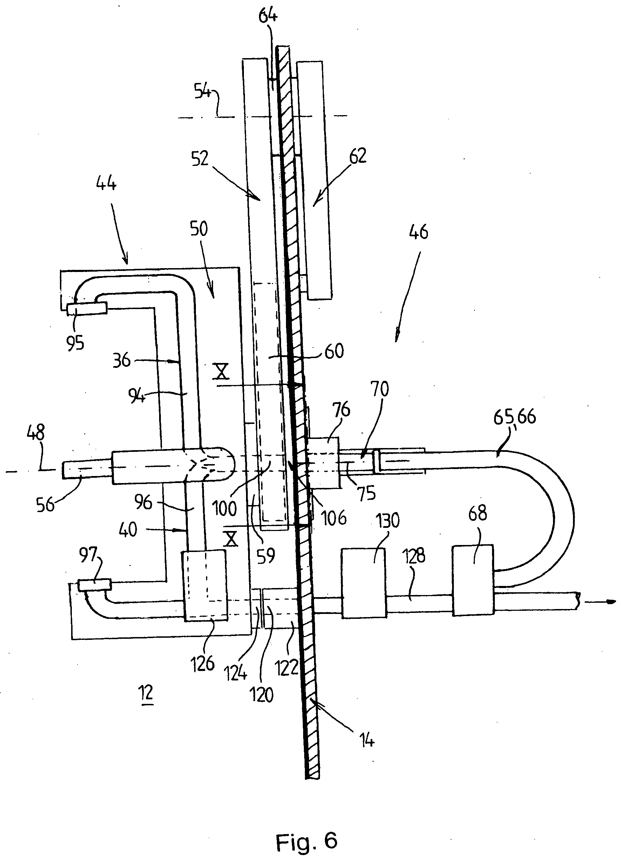

Neben der Öffnung 30 ist am Oberteil 26 eine weitere, durch ein Ventil verschließbare Öffnung (nicht sichtbar) vorgesehen, an der nach der Aufnahme des Behälters 18 in der Vorrichtung 10 eine erste Rohrleitung 36 (Fig. 6) angeflanscht wird, durch die Dampf, Heißwasser, Kaltwasser, Luft, ein Wasser-Luft-Gemisch oder andere flüssige oder gasförmige Behandlungsmedien in den Behälter 18 zu- oder abgeführt bzw. Behandlungsmedien aus dem Behälter 18 verdrängt werden kann. Am tiefsten Punkt des Bodens 24 ist eine noch weitere Öffnung 38 vorgesehen, die ebenfalls durch ein Klappenventil verschließbar ist. Nach der Aufnahme des Behälters 18 in der Vorrichtung 10 kann an dieser Öffnung 38 eine zweite Rohrleitung 40 (Fig. 6) angeflanscht werden, durch die Dampf, Heißwasser, Kaltwasser, Luft, ein Wasser-Luft-Gemisch oder andere flüssige oder gasförmige Behandlungsmedien in den Behälter 18 zugeführt bzw. die durch die Leitung 36 zugeführten Behandlungsmedien aus dem Behälter 18 abgeführt oder verdrängt werden können, so dass die Behandlungsmedien bei der Behandlung durch den Behälter 18 hindurch strömen können. Im Inneren ist der Behälter 18 in einem geringen Abstand über dem Boden 24 mit einem Siebboden (nicht sichtbar) versehen, der bei der Behandlung einen Hindurchtritt der Behandlungsmedien gestattet und bei aufrechtem Behälter 18 ein Abtropfen der Gegenstände erlaubt.

Die Reinigungs- und Sterilisationsvorrichtung 10 umfasst einen innerhalb des Reinraums 12 angeordneten Reinraumteil 44 und einen außerhalb des Reinraums 12 hinter der Begrenzungswand 14 angeordneten, durch die Begrenzungswand 14 vom Reinraumteil 44 getrennten Maschinenteil 46, wie in Fig. 6 dargestellt.

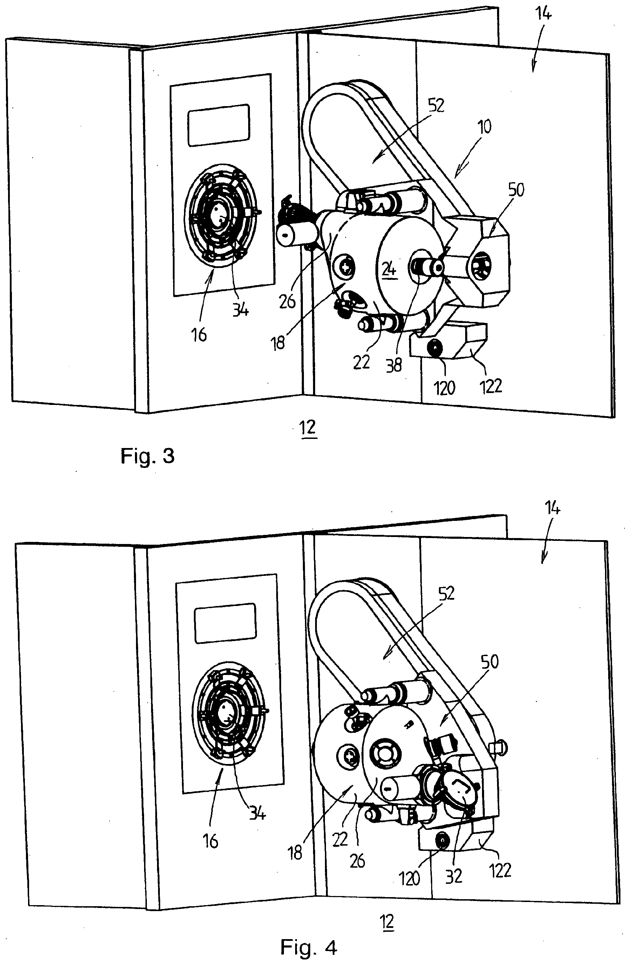

Der Reinraumteil 44 umfasst eine den Behälter 18 aufnehmende Halterung 50, die während der Behandlung zusammen mit dem Behälter 18 motorisch um eine horizontale Schwenkachse 48 schwenkbar ist, um die Gegenstände im Behälter 18 in Bewegung zu versetzen, sowie einen die Halterung 50 tragenden Schwenk- oder Hubarm 52, der motorisch um eine zur Schwenkachse 48 parallele Schwenkachse 54 schwenkbar ist, um die Halterung 50 vor oder nach der Behandlung zum Befüllen oder zum Entleeren des Behälters 18 anzuheben oder abzusenken. Während der Behandlung der kleinen Gegenstände im Behälter 18 wird nur die Halterung 50 zusammen mit dem Behälter 18 um die Schwenkachse 48 verschwenkt, wie in Fig. 3 und 4 dargestellt, während der Schwenk- oder Hubarm 52 in der in Fig. 3 und 4 dargestellten Behandlungsstellung verbleibt, in der er von der Schwenkachse 54 gesehen schräg nach unten weist und die Schwenkachse 48 schräg unterhalb von der Schwenkachse 54 angeordnet ist.

Zur Befestigung des Behälters 18 weist die Halterung 50 zwei parallel zueinander überstehende horizontale Tragdorne 56 auf, die sich in zwei diametral entgegengesetzte zylindrische Aufnahmebuchsen 58 am Behälterunterteil 22 einführen lassen. Weitere Einzelheiten eines zur Behandlung von kleineren Gegenständen geeigneten Behälters sind in der EP 1 449 543 B1 und der EP 1 510 227 B1 beschrieben, auf die diesbezüglich verwiesen wird.

Der Schwenk- oder Hubarm 52 umgibt einen Hohlraum (nicht dargestellt), in dem ein Drehantrieb 60 zum Verschwenken der Halterung 50 um die Schwenkachse 48 untergebracht ist. Der Drehantrieb 60 umfasst einen elektrischen Antriebsmotor und ein selbsthemmendes Untersetzungsgetriebe (nicht dargestellt), zum Beispiel ein Schneckengetriebe, dessen Abtriebswelle sich durch ein zur Schwenkachse 48 koaxiales Drehlager 59 hindurch zur Halterung 50 erstreckt und drehfest mit dieser verbunden ist.

Der Maschinenteil 46 kann u.a. einen Heißwasserbereiter, einen Drucklufterzeuger und einen Dampferzeuger (nicht dargestellt) umfassen, die durch die Rohrleitungen 36, 40 mit dem Behälter 18 verbindbar sind. Weiter umfasst der Maschinenteil 46 einen Drehantrieb 62 zum Verschwenken des Schwenk- oder Hubarms 52, dessen oberes Ende in einem Drehlager 64 der Begrenzungswand 14 gelagert ist.

Wie am besten in Fig. 6, 7 und 8 dargestellt, umfassen die Rohrleitungen 36, 40 auf der vom Reinraum 12 abgewandten Seite der Begrenzungswand 14 jeweils ein flexibles Teilstück 65 bzw. 66, die in der Behandlungsstellung des Schwenk- oder Hubarms 52 in horizontaler Richtung beiderseits von der Schwenkachse 48 angeordnet sind und sich nach hinten von der Begrenzungswand 14 weg, im Bogen nach unten und wieder nach vorne auf die Begrenzungswand 14 zu erstrecken, wie in Fig. 6 dargestellt. Am unteren Ende der beiden Teilstücke 65, 66 ist jeweils ein Wegeventil 68 angeordnet, mit dem sich die Teilstücke 65, 66 wahlweise mit dem Heißwasserbereiter, dem Drucklufterzeuger oder dem Dampferzeuger oder alternativ mit einer tiefer gelegenen Entleerungsöffnung (nicht dargestellt) verbinden lassen, durch welche die gesamte Flüssigkeit aus den beiden Teilstücken 65, 66 sowie aus den reinraumseitigen Teilen der Leitungen 36, 40 allein durch ihrer Schwerkraft abfließen kann. Die flexiblen Teilstücke 65, 66 weisen eine ausreichende Länge auf, so dass ihre reinraumseitigen Enden beim Verschwenken des Behälters aus dessen aufrechter Stellung (Fig. 2) in beide Richtungen (Fig. 3 bzw. 4) der Schwenkbewegung der Halterung 50 folgen können, ohne dass es zum Auftreten von Zugspannungen in den Teilstücken 65, 66 kommt und diese nur minimal auf Torsion beansprucht werden.

Wie am besten in den Figuren 6 bis 8 dargestellt, weisen die beiden Teilstücke 65, 66 an ihrem zum Reinraum 12 benachbarten Ende jeweils einen ersten Rohrleitungsabschnitt 70 auf, der sich parallel zur Schwenkachse 48 geradlinig durch die Wand 14 hindurch erstreckt. Die beiden Rohrleitungsabschnitte 70 sind beiderseits der Schwenkachse 48 im gleichen Abstand von dieser angeordnet. Im Bereich der Begrenzungswand 14 verlaufen die beiden Abschnitte 70 durch einen Schiebekörper 72 mit einem in Bezug zur Schwenkachse 48 unrunden Querschnitt, der wiederum einen Drehkörper 74 durchsetzt. Der Drehkörper 74 weist einen zur Drehachse 75 koaxialen Querschnitt auf und ist in einem starr mit der Begrenzungswand 14 verbundenen zylindrischen Gehäuse 76 um eine Drehachse 75 frei drehbar gelagert. Der Schiebekörper 72 ist innerhalb des Drehkörpers 74 in axialer Richtung der Drehachse 75 verschiebbar und wird wegen seines unrunden Querschnitts beim Verdrehen des Drehkörpers 74 um die Drehachse 75 mitgedreht. Die Drehachse 75 fluchtet mit der Schwenkachse 48, wenn sich der Schwenk- oder Hubarm 52 in der Behandlungsstellung befindet.

Zum Verschieben des Verschiebekörpers 72 in Bezug zum Drehkörper 74 dient ein Hydraulik- oder Pneumatik-Zylinder 78, der auf der vom Reinraum 12 abgewandten Seite der Wand 14 hinter den Körpern 72, 74 und den Rohrleitungsabschnitten 70 angeordnet ist. Der Zylinder 78 umfasst ein Zylinderrohr 80, das über einen Bügel 82 starr mit dem Drehkörper 74 verbunden ist, sowie eine Kolbenstange 84, die zwischen den beiden Rohrleitungsabschnitten 70 am Verschiebekörper 72 angelenkt ist.

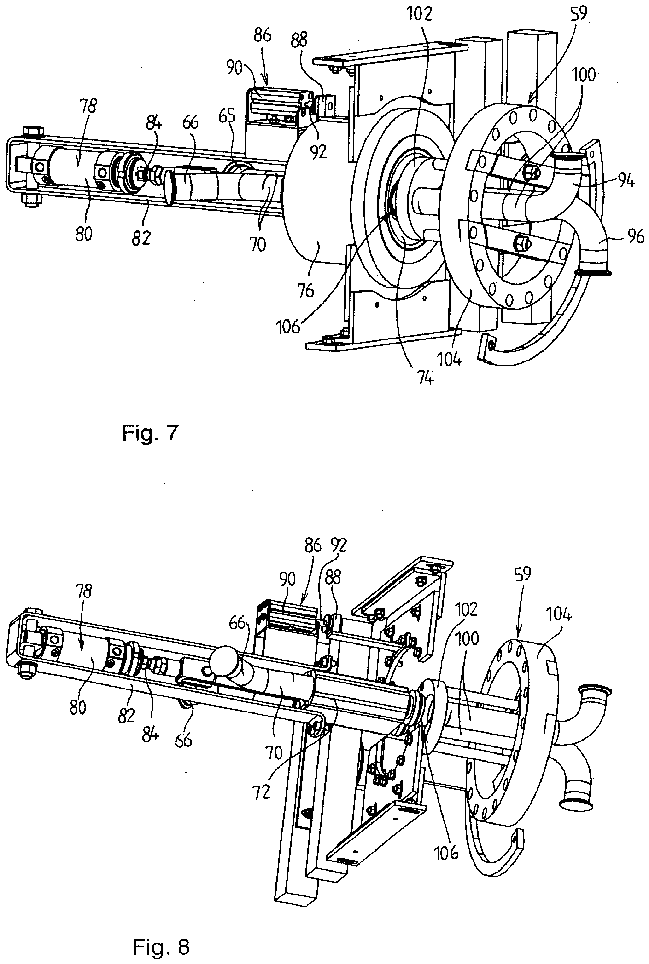

Oberhalb von dem Bügel 82 bzw. vom Gehäuse 76 befindet sich eine Verriegelungseinrichtung 86, die aus einer starr mit dem Gehäuse 76 und der Wand 14 verbundenen Riegelaufnahme 88, einem drehfest mit dem Bügel 82 und über den Bügel 82 mit dem zweiten Körper 74 verbundenen Stellorgan 90, sowie einem mittels des Stellorgans 90 parallel zur Schwenkachse 48 verschiebbaren Riegel 92 besteht. Wenn das Stellorgan 90 ausgefahren ist, greift der Riegel 92 in die Riegelaufnahme 88 ein, wie in Fig. 8 dargestellt. In diesem Zustand wird der zweite Körper 74 drehfest mit dem Gehäuse 76 verriegelt, so dass er sich in Bezug zur Wand 14 nicht drehen kann. Wenn das Stellorgan 90 eingefahren und der Riegel 92 aus der Riegelaufnahme 88 ausgerückt ist, kann sich hingegen der zweite Körper 74 frei im Gehäuse 76 und damit in Bezug zur Wand 14 um die Schwenkachse 48 drehen.

Der Riegel 92 und die Riegelaufnahme 88 besitzen komplementäre konische Oberflächen, die gegeneinander angepresst werden, wenn der Riegel 92 in die Riegelaufnahme 88 eingreift. In diesem Zustand wird so der zweite Körper 74 in einer definierten Drehlage festgehalten, in der die Mittelachsen der beiden Rohrleitungsabschnitte 70 zusammen mit der Schwenkachse 48 eine horizontale Ebene aufspannen.

Der Drehantrieb 62 zum Verschwenken des Schwenk- oder Hubarms 52 besteht im Wesentlichen aus einem an der Rückseite der Begrenzungswand 14 angebrachten elektrischen Antriebsmotor mit selbsthemmendem Untersetzungsgetriebe (nicht dargestellt), zum Beispiel einem Schneckengetriebe, dessen Abtriebswelle sich durch das Drehlager 64 hindurch zum Schwenk- oder Hubarm 52 erstreckt und drehfest mit diesem verbunden ist. Bevorzugt ist der Drehantrieb 62 baugleich mit dem Drehantrieb 60.

Der Reinraumteil 44 umfasst neben dem Schwenk- oder Hubarm 52 und der Halterung 50 jeweils ein durch die Halterung 50 und durch den Schwenk- oder Hubarm 52 hindurch in Richtung der Wand 14 verlaufendes Teilstück 94 bzw. 96 von jeder Rohrleitung 36, 40. Das Teilstück 94 der Rohrleitung 36 erstreckt sich von einem mit der Öffnung des Behälteroberteils 26 verbindbaren Anschlussflansch 95 durch die Halterung 50 hindurch nach unten bis in Höhe der Schwenkachse 48, während sich das Teilstück 96 von einem mit der Öffnung 38 am Behälterboden 24 verbindbaren Anschlussflansch 97 durch die Halterung 50 hindurch nach oben bis in Höhe der Schwenkachse 48 erstreckt. Beide Teilstücke 94, 96 sind dort in Richtung der Wand 14 umgebogen und umfassen jeweils einen zweiten Rohrleitungsabschnitt 100, der sich parallel zur Schwenkachse 48 durch das Drehlager 59 der Halterung 50 hindurch in den Schwenk- oder Hubarm 52 und weiter gerade durch diesen hindurch in Richtung der Wand 14 erstreckt.

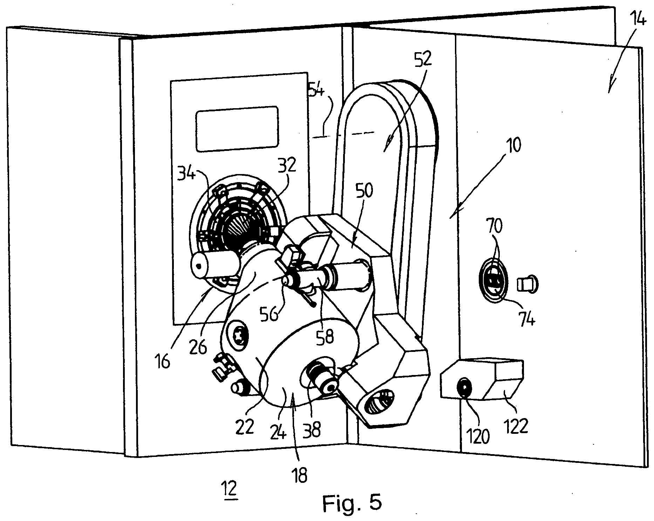

Die beiden ersten Rohrleitungsabschnitte 70 und die beiden zweiten Rohrleitungsabschnitte 100 sind zwischen dem Schwenk- oder Hubarm 52 und der Begrenzungswand 14 mittels einer Rohrleitungskupplung 106 lösbar miteinander verbunden. Die Rohrleitungskupplung 106 kann in der Behandlungsstellung des Schwenk- oder Hubarms 52 geschlossen und geöffnet werden, indem die ersten Rohrleitungsabschnitte 70 mittels des Zylinders 78 parallel zur Schwenkachse 48 auf den Schwenk- oder Hubarm 52 zu oder von diesem weg verschoben werden, bis bei geschlossener Kupplung 106 die Enden der jeweils gegenüberliegenden Rohrleitungsabschnitte 70, 100 dichtend gegeneinander angepresst werden bzw. bei geöffneter Kupplung 106 eine solchen Abstand aufweisen, dass sie sich beim Verschwenken des Schwenk- oder Hubarms 52 berührungsfrei aneinander vorbei bewegen lassen. In diesem Zustand sind die Rohrleitungsabschnitte 70 so weit in Richtung der Begrenzungswand 14 zurückgezogen, dass sie nicht mehr über diese überstehen, wie in Fig. 5 dargestellt.

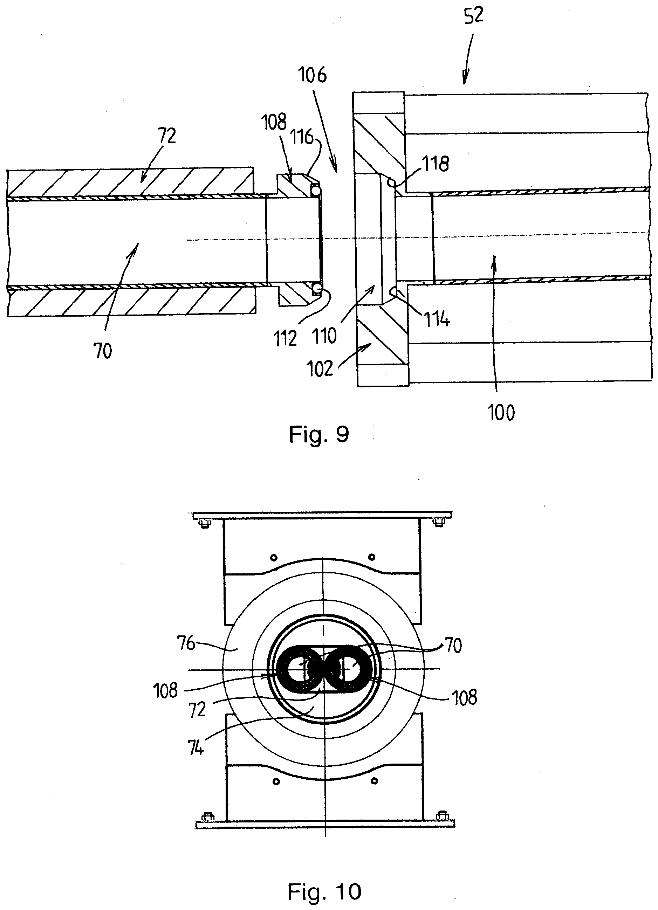

Wie am besten in Fig. 8 und 9 dargestellt, münden die der Wand 14 zugewandten Enden der beiden Rohrleitungsabschnitte 100 in ein platten- oder scheibenförmiges Kupplungselement 102 der Rohrleitungskupplung 106, das die Rohrleitungsabschnitte 100 starr miteinander und mit einem drehfest mit der Halterung 50 verbundenen Innenring 104 des Drehlagers 59 verbindet.

Wie am besten in Fig. 9 dargestellt, ist jeder der beiden ersten Rohrleitungsabschnitte 70 an seinem dem Schwenk- oder Hubarm 52 zugewandten Ende mit einem Kupplungselement 108 der Rohrleitungskupplung 106 versehen, das nach dem Schließen der Kupplung 106 in eine gegenüberliegende komplementäre Vertiefung 110 des Kupplungselements 102 ragt und für eine formschlüssige und drehfeste Verbindung zwischen den Rohrleitungsabschnitten 70 und 100 beim Kuppeln sorgt.

Die Kupplungselemente 108 weisen an ihren freien Enden jeweils eine in eine Ringnut eingesetzten Dichtung 112 auf, die beim Schließen der Kupplung 106 gegen eine parallele Dichtfläche 114 in der gegenüberliegenden Vertiefung 110 angepresst wird und für eine dichte Verbindung der Rohrleitungsabschnitte 70, 100 sorgt. Die Kupplungselemente 108 weisen weiter eine konisch verjüngte Umfangsfläche 116 auf, die beim Schließen der Kupplung 106 mit einer komplementären konischen Fläche 118 in der gegenüberliegenden Vertiefung 110 zusammenwirkt und für eine gegenseitige Zentrierung der beiden Rohrleitungsabschnitte 70, 100 sorgt, so dass die Mittelachse von jedem der Rohrleitungsabschnitte 70 genau mit der Mittelachse von einem gegenüberliegenden Rohrleitungsabschnitt 100 fluchtet.

Wenn in der Behandlungsstellung die Halterung 50 mit dem Behälter 18 mittels des Drehantriebs 60 in Bezug zum Schwenk- oder Hubarm 52 um die Schwenkachse 48 geschwenkt wird, wie in Fig. 3 und 4 dargestellt, werden die zuvor an den Behälter 18 angekuppelten reinraumseitigen Teilstücke 94, 96 der Leitung 36 einschließlich der beiden durch den Schwenk- oder Hubarm 52 hindurch verlaufenden zweiten Rohrleitungsabschnitte 100 um die Schwenkachse 48 mitgeschwenkt. Bei geschlossener Kupplung 106 werden infolge der drehfesten Verbindung der gegenüberliegenden Rohrleitungsabschnitte 70, 100 somit auch die beiden ersten Rohrleitungsabschnitte 70 und damit die maschinenseitigen Teilstücke 65, 66 der Rohrleitungen 36, 40 mitgeschwenkt.

Zum Öffnen der Kupplung 106 wird die Halterung 50 mit dem Behälter 18 in die in Fig. 2 dargestellte aufrechte Stellung geschwenkt, in der die Mittelachsen der Rohrleitungsabschnitte 70, 100 mit der Schwenkachse 48 eine horizontale Ebene aufspannen. Bevor die Kupplung 106 geöffnet wird, wird die Verriegelungseinrichtung 86 betätigt, um den Riegel 92 in die Riegelaufnahme 88 einzurücken und bei geöffneter Kupplung 106 ein Verdrehen der beiden Körper 72, 74 in Bezug zur Wand 14 zu verhindern. Nachdem dann die Kupplung 106 durch Zurückziehen des Schiebekörpers 72 und der ersten Rohrleitungsabschnitte 70 geöffnet worden ist, kann der Schwenk- oder Hubarm 52 frei verschwenkt werden. In diesem Zustand kann der Behälter 18 durch Verschwenken des Schwenk- oder Hubarms 52 und/oder der Halterung 50 zum Beispiel zur direkten Befüllung aus der Befüllstation an die Andocköffnung 16 angedockt werden, wie in Fig. 5 dargestellt, oder zur Entleerung in einen anderen Behälter oder eine Übergabestation umgedreht werden, so dass die Öffnung 30 nach unten weist.

Der Reinraumteil 44 umfasst weiter einen Entleerungsanschluss 120, der unterhalb vom unteren Ende des Schwenk- oder Hubarms 52 in einem über die Begrenzungswand 14 in den Reinraum 12 überstehenden Vorsprung 122 angeordnet ist, wie am besten in Fig. 3 und 4 dargestellt. Der Anschluss 120 dient zur Restentleerung des Behälters 18 in seiner in Fig. 1 und 2 dargestellten aufrechten Stellung. In dieser Stellung lässt sich der Entleerungsanschluss 120 flüssigkeits- und gasdicht mit einer gegenüberliegenden Austrittsöffnung 124 der Halterung 50 verbinden.

Der Entleerungsanschluss 120 und die Austrittsöffnung 124 liegen tiefer als die untere Behälteröffnung 38, so dass im Behälter 18 und im Teilstück 96 der Rohrleitung 40 befindliches flüssiges Behandlungsmedium durch Schwerkraft vollständig in den Entleerungsanschluss 120 und von dort in eine in den Maschinenteil 46 führende Rohrleitung 128 abläuft, wenn die Austrittsöffnung 124 in der aufrechten Stellung des Behälters 18 mit dem Entleerungsanschluss 120 verbunden ist, während ein hinter der Wand 14 in der Leitung 128 angeordnetes Ventil 130 und ein vor der Austrittsöffnung 124 angeordnetes Wegeventil 126 zur Austrittsöffnung 124 hin geöffnet sind.

Im Betrieb wird der Behälter 18 im Reinraum 12 mittels des Transportwagens in aufrechter Stellung in die Halterung 50 eingesetzt, wie in Fig. 1 und 2 dargestellt, wobei sich der Schwenk- oder Hubarm 52 in der Behandlungsstellung befindet. Dann werden die Teilstücke 94, 96 der Rohrleitungen 36, 40 an den Behälter 18 angekuppelt und die Kupplung 106 geöffnet. Anschließend werden sowohl der Schwenk- oder Hubarm 52 und die Halterung 50 verschwenkt, um die Öffnung 30 des Behälters 18 zuerst an die Befüllöffnung 16 anzunähern und sie dann mittels des Alpha- und Beta-Teils 34, 32 des Rapid-Transfer-Ports ohne die Gefahr einer Kontamination des Reinraums 12 direkt an die Befüllöffnung 16 anzudocken, wie in Fig. 5 dargestellt. Damit die Gegenstände allein durch ihre Schwerkraft in den Behälter 18 rutschen, wird der Behälter 18 durch Verschwenken des Schwenk- oder Hubarms 52 und der Halterung 50 zum Befüllen in eine schräg geneigte Ausrichtung unterhalb der Befüllöffnung 16 gebracht.

Nach dem Schließen der Öffnung 30 mittels des Klappenventils 28 werden der Alpha- und Beta-Teil 34, 32 des Rapid-Transfer-Ports wieder voneinander getrennt und dann einerseits der Schwenk- oder Hubarm 52 in die Behandlungsstellung und andererseits der Behälter 18 in die aufrechte Stellung zurück geschwenkt, wie in Fig. 2 dargestellt. In der Behandlungsstellung wird zum einen die Kupplung 106 geschlossen, indem die Kolbenstange 84 des Zylinders 78 ausgefahren wird, um den Körper 72 in Richtung des Schwenk- oder Hubarms zu bewegen und die Kupplungsstücke 108 in die gegenüberliegenden Vertiefungen 110 der Kupplungsplatte 102 einzurücken und dadurch die Rohrleitungsabschnitte 70, 100 drehfest und flüssigkeitsdicht zu verbinden. Zum anderen wird der Riegel 92 der Verriegelungseinrichtung 86 mittels des Stellorgans 90 aus der Riegelaufnahme 88 heraus bewegt, so dass die Körper 72 und 74 frei drehbar sind und damit die Rohrleitungsabschnitte 70 während der Behandlung mit der Halterung 50 mit bewegt werden können.

Während der nachfolgenden Behandlung der Gegenstände im Behälter 18 können durch die Leitungen 36, 40 aus dem Maschinenteil 46 wahlweise Heißwasser, ein Wasser-Luft-Gemisch oder ein anderes Behandlungsmedium durch den Behälter 18 umgewälzt werden, um die darin befindlichen Gegenstände zu reinigen und anhaftende Fremdkörper abzuspülen. Gleichzeitig wird der Behälter 18 durch Verschwenken der Halterung 50 mittels des Drehantriebs 60 um die Schwenkachse 48 in Bewegung versetzt, wie in Fig. 2 und 3 dargestellt, um die Reinigungswirkung zu verbessern und insbesondere bei der Reinigung von Verschlüssen aus Gummi ein Verklumpen zu verhindern. Während der Behandlung bleibt der Schwenk- oder Hubarm 52 in der Behandlungsstellung.

Um den Behälter 18 und die Rohrleitungen 36, 40 vor einer anschließenden Sterilisation der Gegenstände vollständig vom Restwasser zu entleeren, wird zuerst der Behälter 18 in seine aufrechte Stellung gebracht und die Austrittsöffnung 124 mit dem Entleerungsanschluss 120 verbunden, bevor das Wegeventil 126 nacheinander so angesteuert wird, dass das noch im Behälter 18 bzw. im Teilstück 96 der Leitung 40 befindliche Wasser allein durch Schwerkraft in den Entleerungsanschluss 120 strömt und dabei eventuelle abgereinigte Partikel mit sich führt.

Nach dem Schließen der Austrittsöffnung 124 mittels des Wegeventils wird zur Sterilisation der im Behälter 18 befindlichen Gegenstände Sattdampf durch die Rohrleitung 40 in den Behälter 18 und nach seinem Hindurchtritt durch den Behälter 18 durch die Rohrleitung 36 in den Maschinenteil 46 zurück geführt, wobei die Halterung 50 und der Behälter 18 vorteilhaft ebenfalls in eine Schwenkbewegung versetzt werden, wie in Fig. 2 und 3 dargestellt.

Die gereinigten und sterilisierten Gegenstände können aus dem Behälter 18 ausgetragen werden, indem die Halterung 50 mit dem Behälter 18 in eine Stellung geschwenkt wird, in der die Öffnung 30 schräg nach unten weist. Der Behälter kann in dieser Stellung in einen anderen Behälter (nicht dargestellt) oder direkt in eine Andocköffnung einer Übergabestation (nicht dargestellt) entleert werden, wenn diese sich im Schwenkbereich der Halterung 18 und/oder des Schwenk- oder Hubarms 52 befindet.

## Patentansprüche

1. Vorrichtung zur Behandlung von kleineren Gegenständen, insbesondere zur Reinigung und Sterilisation von Spritzenkolben oder Verschlüssen von Spritzen, Infusionsflaschen und Vials für pharmazeutische Zwecke, mit einem Behälter (18) zur Aufnahme einer Mehrzahl der Gegenstände, einer Halterung (50), die gemeinsam mit dem Behälter (18) motorisch um eine horizontale Schwenkachse (48) schwenkbar ist, sowie mindestens zwei vom Behälter (18) aus durch die Halterung (50) und eine ortsfeste Wand (14) verlaufenden Rohrleitungen (36, 40) zur Umwälzung mindestens eines Behandlungsmediums durch den Behälter (18), **dadurch gekennzeichnet, dass** die Halterung (50) an einem Schwenk- oder Hubarm (52) angebracht ist, der in Bezug zur Wand (14) motorisch um eine zur Schwenkachse (48) parallele zweite Schwenkachse (54) schwenkbar ist, und dass die Rohrleitungen (36, 40) durch den Schwenk- oder Hubarm (52) hindurch verlaufen und zwischen der Wand (14) und dem Schwenk- oder Hubarm (52) mit einer lösbaren Rohrleitungskupplung (106) versehen sind.

2. Vorrichtung nach Anspruch 1, **dadurch gekennzeichnet, dass** durch die Wand (14) verlaufende erste Abschnitte (70) der Rohrleitungen (36, 40) bei geschlossener Rohrleitungskupplung (106) drehfest mit zweiten Abschnitten (100) der Rohrleitungen (36, 40) gekuppelt sind, die mit dem Behälter (18) und der Halterung (50) motorisch um die Schwenkachse (48) geschwenkt werden.

3. Vorrichtung nach Anspruch 1 oder 2, **dadurch gekennzeichnet, dass** die Rohrleitungskupplung (106) zwischen zwei durch die Wand (14) verlaufenden ersten Abschnitten (70) und zwei durch den Schwenk- oder Hubarm (52) verlaufenden zweiten Abschnitten (100) der Rohrleitungen (36, 40) angeordnet ist.

4. Vorrichtung nach Anspruch 2 oder 3, **dadurch gekennzeichnet, dass** die ersten Abschnitte (70) der Rohrleitungen (36, 40) in Bezug zur Wand (14) um eine horizontale Drehachse (75) drehbar sind, die in einer Stellung des Schwenk- oder Hubarms (52) mit der Schwenkachse (48) fluchtet.

5. Vorrichtung nach einem der Ansprüche 2 bis 4, **dadurch gekennzeichnet, dass** die ersten Abschnitte (70) der Rohrleitungen (36, 40) in Bezug zur Wand (14) in einer zur Schwenkachse (48) parallelen Richtung auf die zweiten Abschnitte (100) der Rohrleitungen (36, 40) zu und von diesen weg bewegbar sind.

6. Vorrichtung nach einem der Ansprüche 2 bis 5, **dadurch gekennzeichnet, dass**.Mittelachsen der ersten und der zweiten Abschnitte (70; 100) der Rohrleitungen (36, 40) in gleichen radialen Abständen von der Drehachse (75) bzw. von der Schwenkachse (48) angeordnet sind.

7. Vorrichtung nach einem der Ansprüche 2 bis 6, **dadurch gekennzeichnet, dass** die ersten und die zweiten Abschnitte (70; 100) der Rohrleitungen (36, 40) beim Schließen der Rohrleitungskupplung (106) formschlüssig und in Bezug zur Schwenkachse (48) drehfest miteinander in Eingriff treten.

8. Vorrichtung nach einem der Ansprüche 2 bis 7, **dadurch gekennzeichnet, dass** die Enden der ersten Abschnitte (70) der Rohrleitungen (36, 40) beim Schließen der Rohrleitungskupplung (106) in gegenüberliegende komplementäre Vertiefungen (110) an den Enden der zweiten-Abschnitte (100) eintreten, oder umgekehrt.

9. Vorrichtung nach einem der Ansprüche 2 bis 8, **gekennzeichnet durch** eine Drehverriegelungseinrichtung (86), welche die ersten Abschnitte (70) der Rohrleitungen (36, 40) in einer definierten Drehstellung in Bezug zur Wand (14) festhält, wenn die ersten und die zweiten Abschnitte (70, 100) bei geöffneter Rohrleitungskupplung (106) voneinander getrennt sind.

10. Vorrichtung nach einem der vorangehenden Ansprüche, **dadurch gekennzeichnet, dass** der Schwenk- oder Hubarm (52) in der Nähe von seinem einen Ende durch ein Drehgelenk (64) mit der Wand (14) verbunden ist und in der Nähe von seinem anderen Ende die Halterung (50) trägt, die durch ein Drehgelenk (59) mit dem Schwenk- oder Hubarm (52) verbunden ist.

11. Vorrichtung nach einem der vorangehenden Ansprüche, **gekennzeichnet durch** einen jenseits der Wand (14) angeordneten Drehantrieb (62) zum Verschwenken des Schwenk- oder Hubarms (52) und einem im Inneren des Schwenk- oder Hubarms (52) angeordneten Drehantrieb (60) zum Verschwenken der Halterung (50).

12. Vorrichtung nach einem der vorangehenden Ansprüche, **dadurch gekennzeichnet, dass** die Wand (14) eine Begrenzungswand eines Reinraums (12) ist und dass der Schwenk- oder Hubarm (52), die Halterung (50) und der Behälter (18) innerhalb des Reinraums (12) und ein Maschinenteil (46) sowie mindestens eine Quelle für das Behandlungsmedium jenseits der Wand (14) und außerhalb des Reinraums (12) angeordnet sind.

13. Vorrichtung nach einem der Ansprüche 2 bis 12, **dadurch gekennzeichnet, dass** der Schwenk- oder Hubarm (52) zwischen der Wand (14) und der Halterung (50) angeordnet ist und dass die zweiten Abschnitte (100) der Rohrleitungen (36, 40) bei geschlossener Rohrleitungskupplung (106) entlang der Schwenkachse (48) des Behälters (18) zur Wand und zu den gegenüberliegenden ersten Abschnitten (70) der Rohrleitungen (36, 40) verlaufen.

14. Vorrichtung nach einem der Ansprüche 2 bis 13, **dadurch gekennzeichnet, dass** die durch die Wand (14) verlaufenden ersten Abschnitte (70) der Rohrleitungen (36, 40) bei geschlossener Rohrleitungskupplung (106) nicht über eine der Halterung (50) zugewandte Oberfläche der Wand (14) überstehen.

## Claims

1. Device for treating small objects, in particular for cleaning and sterilizing syringe plungers or stoppers of syringes, infusion bottles and vials for pharmaceutical purposes, having a container (18) for receiving a plurality of the objects, a holder (50) which, together with the container (18), can be pivoted by a motor about a horizontal pivot axis (48), and at least two pipes (36, 40) which extend from the container (18) through the holder (50) and through a fixed wall (14) and which are used for circulating at least one treatment medium through the container (18), **characterized in that** the holder (50) is mounted on a pivoting or lifting arm (52) which, with respect to the wall (14), can be pivoted by a motor about a second pivot axis (54) parallel to the pivot axis (48), and **in that** the pipes (36, 40) extend through the pivoting or lifting arm (52) and are provided, between the wall (14) and the pivoting or lifting arm (52), with a releasable pipe coupling (106).

2. Device according to Claim 1, **characterized in that**, when the pipe coupling (106) is closed, first portions (70) of the pipes (36, 40) extending through the wall (14) are coupled to second portions (100) of the pipes (36, 40) for conjoint rotation therewith, which second portions (100) are pivoted by a motor about the pivot axis (48) together with the container (18) and the holder (50).

3. Device according to Claim 1 or 2, **characterized in that** the pipe coupling (106) is arranged between two first portions (70) of the pipes (36, 40) extending through the wall (14) and two second portions (100) of the pipes (36, 40) extending through the pivoting or lifting arm (52).

4. Device according to Claim 2 or 3, **characterized in that** the first portions (70) of the pipes (36, 40) are rotatable, with respect to the wall (14), about a horizontal rotation axis (75), which is in alignment with the pivot axis (48) in one position of the pivoting or lifting arm (52).

5. Device according to one of Claims 2 to 4, **characterized in that**, with respect to the wall (14), the first portions (70) of the pipes (36, 40) are movable towards and away from the second portions (100) of the pipes (36, 40) in a direction parallel to the pivot axis (48).

6. Device according to one of Claims 2 to 5, **characterized in that** central axes of the first and of the second portions (70; 100) of the pipes (36, 40) are arranged at the same radial distances from the rotation axis (75) and from the pivot axis (48), respectively.

7. Device according to one of Claims 2 to 6, **characterized in that**, when the pipe coupling (106) is closed, the first and the second portions (70; 100) of the pipes (36, 40) come into form-fit engagement with each other for conjoint rotation with respect to the pivot axis (48).

8. Device according to one of Claims 2 to 7, **characterized in that**, when the pipe coupling (106) is closed, the ends of the first portions (70) of the pipes (36, 40) engage in opposite, complementary depressions (110) at the ends of the second portions (100) or *vice versa.*

9. Device according to one of Claims 2 to 8, **characterized by** a rotation-locking mechanism (86) which secures the first portions (70) of the pipes (36, 40) in a defined rotation position with respect to the wall (14) when the first and the second portions (70, 100) are separated from each other when the pipe coupling (106) is opened.

10. Device according to one of the preceding claims, **characterized in that** the pivoting or lifting arm (52), near one end thereof, is connected to the wall (14) by a rotary joint (64) and, near its other end, carries the holder (50), which is connected to the pivoting or lifting arm (52) by a rotary joint (59).

11. Device according to one of the preceding claims, **characterized by** a rotary drive (62) arranged on the other side of the wall (14) and serving to pivot the pivoting or lifting arm (52), and a rotary drive (60) arranged in the interior of the pivoting or lifting arm (52) and serving to pivot the holder (50).

12. Device according to one of the preceding claims, **characterized in that** the wall (14) is a boundary wall of a clean room (12), and **in that** the pivoting or lifting arm (52), the holder (50) and the container (18) are arranged inside the clean room (12), and a machine part (46) and at least one source of the treatment medium are arranged on the other side of the wall (14) and outside the clean room (12).

13. Device according to one of Claims 2 to 12, **characterized in that** the pivoting or lifting arm (52) is arranged between the wall (14) and the holder (50), and **in that**, when the pipe coupling (106) is closed, the second portions (100) of the pipes (36, 40) extend along the pivot axis (48) of the container (18) to the wall and to the opposite first portions (70) of the pipes (36, 40).

14. Device according to one of Claims 2 to 13, **characterized in that**, when the pipe coupling (106) is closed, the first portions (70) of the pipes (36, 40) extending through the wall (14) do not protrude beyond a surface of the wall (14) directed towards the holder (50).

## Revendications

1. Dispositif pour le traitement de petits objets, en particulier pour nettoyer et stériliser des pistons de seringues ou des fermetures de seringues, des flacons de perfusion et des fioles à usage pharmaceutique, comprenant un récipient (18) destiné à recevoir une pluralité des objets, une fixation (50) qui peut pivoter conjointement avec le récipient (18) au moyen d'un moteur autour d'un axe de pivotement horizontal (48), ainsi qu'au moins deux conduites tubulaires (36, 40) s'étendant depuis le récipient (18) à travers la fixation (50) et à travers une paroi fixe (14) pour mettre en circulation au moins un milieu de traitement à travers le récipient (18), **caractérisé en ce que** la fixation (50) est montée sur un bras pivotant ou de levage (52) qui peut pivoter par rapport à la paroi (14) au moyen d'un moteur autour d'un deuxième axe de pivotement (54) parallèle à l'axe de pivotement (48), et **en ce que** les conduites tubulaires (36, 40) s'étendent à travers le bras pivotant ou de levage (52) et sont pourvues, entre la paroi (14) et le bras pivotant ou de levage (52), d'un accouplement de conduites tubulaires détachable (106).

2. Dispositif selon la revendication 1, **caractérisé en ce que** des premières portions (70) des conduites tubulaires (36, 40) s'étendant à travers la paroi (14) lorsque l'accouplement de conduites tubulaires (106) est fermé, sont accouplées de manière solidaire en rotation à des deuxièmes portions (100) des conduites tubulaires (36, 40), qui sont pivotées au moyen d'un moteur autour de l'axe de pivotement (48) avec le récipient (18) et la fixation (50).

3. Dispositif selon la revendication 1 ou 2, **caractérisé en ce que** l'accouplement de conduites tubulaires (106) est disposé entre deux premières portions (70) des conduites tubulaires s'étendant à travers la paroi (14) et deux deuxièmes portions (100) des conduites tubulaires (36, 40) s'étendant à travers le bras pivotant ou de levage (52).

4. Dispositif selon la revendication 2 ou 3, **caractérisé en ce que** les premières portions (70) des conduites tubulaires (36, 40) peuvent tourner par rapport à la paroi (14) autour d'un axe de rotation horizontal (75) qui est aligné avec l'axe de pivotement (48) dans une position du bras pivotant ou de levage (52).

5. Dispositif selon l'une quelconque des revendications 2 à 4, **caractérisé en ce que** les premières portions (70) des conduites tubulaires (36, 40) peuvent être déplacées par rapport à la paroi (14) dans une direction parallèle à l'axe de pivotement (48) vers les deuxièmes portions (100) des conduites tubulaires (36, 40) et à l'écart de celles-ci.

6. Dispositif selon l'une quelconque des revendications 2 à 5, **caractérisé en ce que** les axes médians des premières et des deuxièmes portions (70 ; 100) des conduites tubulaires (36, 40) sont disposés à des distances radiales identiques de l'axe de rotation (75) respectivement de l'axe de pivotement (48).

7. Dispositif selon l'une quelconque des revendications 2 à 6, **caractérisé en ce que** les premières et les deuxièmes portions (70 ; 100) des conduites tubulaires (36, 40), lors de la fermeture de l'accouplement de conduites tubulaires (106), viennent en prise les unes avec les autres par engagement par correspondance de formes et de manière solidaire en rotation par rapport à l'axe de pivotement (48).

8. Dispositif selon l'une quelconque des revendications 2 à 7, **caractérisé en ce que** les extrémités des premières portions (70) des conduites tubulaires (36, 40), lors de la fermeture de l'accouplement de conduites tubulaires (106), entrent dans des renfoncements complémentaires opposés (110) au niveau des extrémités des deuxièmes portions (100), ou inversement.

9. Dispositif selon l'une quelconque des revendications 2 à 8, **caractérisé par** un dispositif de verrouillage de rotation (86) qui fixe les premières portions (70) des conduites tubulaires (36, 40) dans une position de rotation définie par rapport à la paroi (14), lorsque les premières et deuxièmes portions (70, 100) sont séparées les unes des autres lorsque l'accouplement de conduites tubulaires (106) est ouvert.

10. Dispositif selon l'une quelconque des revendications précédentes, **caractérisé en ce que** le bras pivotant ou de levage (52) est connecté à la paroi (14) à proximité de l'une de ses extrémités par une articulation pivotante (64) et à proximité de son autre extrémité porte la fixation (50) qui est connectée par une articulation pivotante (59) au bras pivotant ou de levage (52).

11. Dispositif selon l'une quelconque des revendications précédentes, **caractérisé par** un entraînement en rotation (62) disposée au-delà de la paroi (14) pour faire pivoter le bras pivotant ou de levage (52) et un entraînement en rotation (60) disposé à l'intérieur du bras pivotant de levage (52) pour faire pivoter la fixation (50).

12. Dispositif selon l'une quelconque des revendications précédentes, **caractérisé en ce que** la paroi (14) est une paroi de limitation d'une salle propre (12) et **en ce que** le bras pivotant ou de levage (52), la fixation (50) et le récipient (18) sont disposés à l'intérieur de la salle propre (12) et une pièce de machine (46) ainsi qu'au moins une source pour le fluide de traitement sont disposées au-delà de la paroi (14) et à l'extérieur de la salle propre (12).

13. Dispositif selon l'une quelconque des revendications 2 à 12, **caractérisé en ce que** le bras pivotant ou de levage (52) est disposé entre la paroi (14) et la fixation (50) et **en ce que** les deuxièmes portions (100) des conduites tubulaires (36, 40), lorsque l'accouplement de conduites tubulaires est fermé (106), s'étendent le long de l'axe de pivotement (48) du récipient (18) jusqu'à la paroi et jusqu'aux premières portions opposées (70) des conduites tubulaires (36, 40).

14. Dispositif selon l'une quelconque des revendications précédentes 2 à 13, **caractérisé en ce que** les premières portions (70) des conduites tubulaires (36, 40) s'étendant à travers la paroi (14), lorsque l'accouplement de conduites tubulaires (106) est fermé, ne dépassent pas au-delà d'une surface de la paroi (14) tournée vers la fixation (50).
